(19) Europäisches Patentamt
European Patent Office
Office européen des brevets

(11) Veröffentlichungsnummer: **0 685 741 A1**

# (12) EUROPÄISCHE PATENTANMELDUNG

(21) Anmeldenummer: **95107934.2**

(51) Int. Cl.[6]: **G01N 33/92**

(22) Anmeldetag: **24.05.95**

(30) Priorität: **30.05.94 IT MI941115**

(43) Veröffentlichungstag der Anmeldung:
**06.12.95 Patentblatt 95/49**

(84) Benannte Vertragsstaaten:
**DE FR GB**

(71) Anmelder: **Moschettini, Giovanni Filippo, Dr.**
**Via Casati, 2**
**I-22055 Merate (IT)**
Anmelder: **Carozzi, Angelo Carlo**
**Via Tre Re, 22**
**I-22050 Verderio Inferiore (IT)**
Anmelder: **Orsenigo, Laura**
**Via S. Francesco, 3**
**I-22055 Merate (IT)**
Anmelder: **Magni, Luisa**
**Via Belfiore, 3**
**I-22056 Olgiate Molgora (IT)**
Anmelder: **Mancosu, Mario**
**Via Papa Giovanni XXIII**
**I-22059 Robbiate (IT)**

(72) Erfinder: **Moschettini, Giovanni Filippo, Dr.**
**Via Casati, 2**
**I-22055 Merate (IT)**
Erfinder: **Carozzi, Angelo Carlo**
**Via Tre Re, 22**
**I-22050 Verderio Inferiore (IT)**
Erfinder: **Orsenigo, Laura**
**Via S. Francesco, 3**
**I-22055 Merate (IT)**
Erfinder: **Magni, Luisa**
**Via Belfiore, 3**
**I-22056 Olgiate Molgora (IT)**
Erfinder: **Mancosu, Mario**
**Via Papa Giovanni XXIII**
**I-22059 Robbiate (IT)**

(74) Vertreter: **Jaumann, Paolo**
**Studio Brevetti Jaumann S.n.c.**
**Piazza Castello, 2**
**I-20121 Milano (IT)**

## (54) Verfahren zur Bestimmung von HDL-Cholesterin.

(57) Verfahren zur Bestimmung des HDL-Cholesterins unter Verwendung eines gegenüber dem Luftdruck einen negativen Innendruck aufweisenden Probeglases, das eine entsprechende Menge einer oder mehrerer, Fraktionen des Blutcholesterins niederschlagender Substanzen enthält.

EP 0 685 741 A1

Stand der Technik

Das derzeit bekannte Verfahren zur Bestimmung des HDL-Cholesterins lässt sich wie folgt schematisch darstellen:

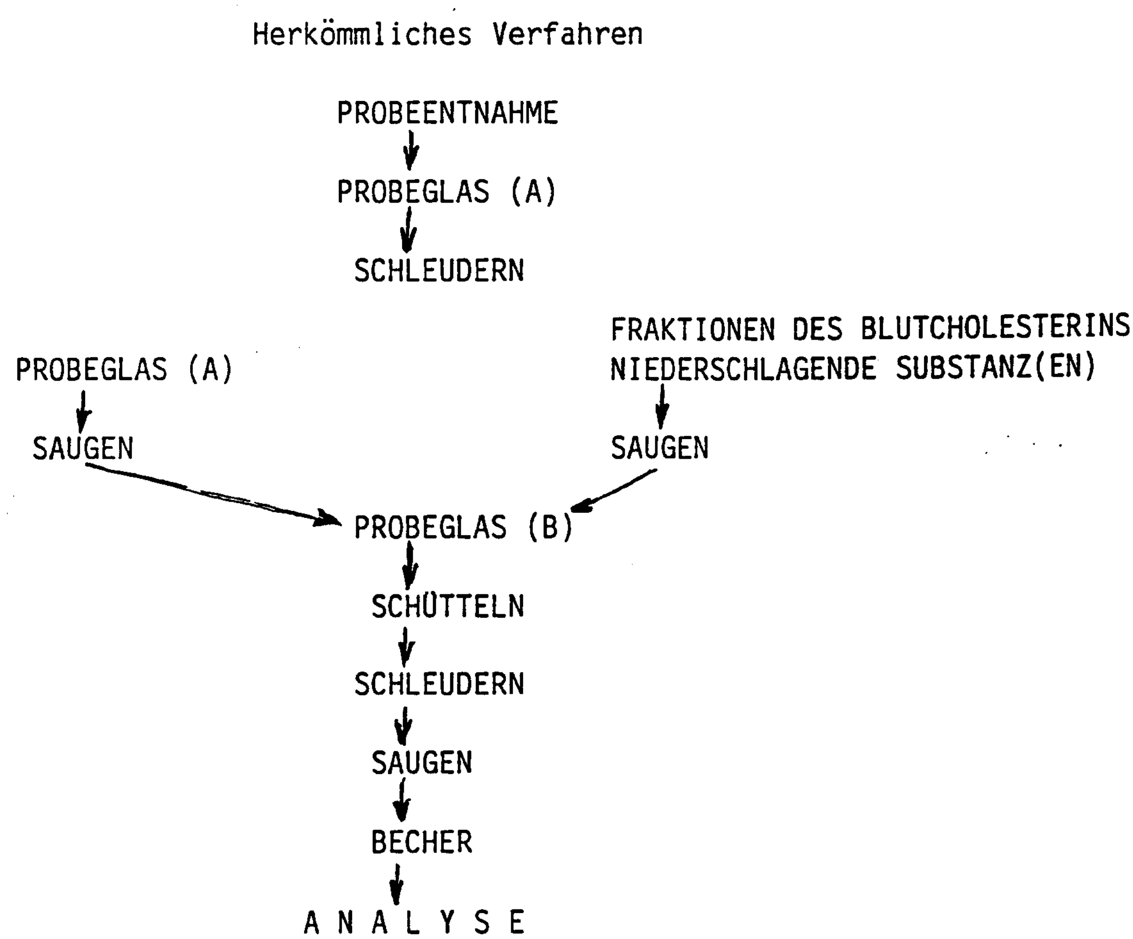


Diesem Schema ist entnehmbar, dass das Verfahren kompliziert ist und erhebliche Nachteile aufweist, die folgendermassen zusammenfassbar sind:

a) mögliche Personenverwechslung bei der Überführung des Plasmas vom Probeglas A in das Probeglas B;

b) weitgehend erforderlicher Zeit-, Material- und Arbeitsaufwand;

c) keine Möglichkeit, die Untersuchung um mehr als 60 Minuten aufzuschieben.

Der Erfindung liegt daher die Aufgabe zugrunde, die aufgezeigten Nachteile unter Verwendung eines neuartigen Verfahrens mit Hilfe eines gegenüber dem Luftdruck einen negativen Innendruck aufweisenden Probeglases zu vermeiden, das eine entsprechende Menge einer oder mehrerer, Fraktionen des Blutcholesterins niederschlagender Substanzen enthält, so dass es als primäres Probeglas verwendbar ist.

Die vorgeschlagene Erfindung besteht darin, bei einer venösen Blutentnahme einen Anteil Vollblut in ein früher vorbereitetes Probeglas aufzunehmen, das nach Zentrifugieren der Bestimmung des HDL-Cholesterins ohne sonstige Behandlungen zugeführt wird.

Bei der Herstellung von Probegläsern mit einem gegenüber dem Luftdruck negativen Innendruck werden in entsprechender Konzentration und Menge, als einziger Bestandteil oder in Verbindung mit weiteren Bestandteilen, eine oder mehrere Substanzen darin eingebracht, die an sich als Fraktionen des Blutcholesterins mittels Schleudern niederschlagende Substanzen bekannt sind.

Als Fraktionen des Blutcholesterins mittels Schleudern niederschlagende Substanzen sind nachstehende Substanzen zu nennen:

a) Heparin-$Mn^{++}$ und dergleichen,

b) Dextransulfat-$Mg^{++}$ und dergleichen,

c) Wolframphosphat-$Mg^{++}$ und dergleichen,

d) Polyethylenglycol und dergleichen.

Derartige Substanze können verschiedentlich miteinander kombiniert sein.

Die Bestimmung des HDL-Cholesterins wurde beispielsweise auf einem Analysator Synchron CX4 (Beckman, Brea, Californie, USA) nach der klassischen Methode der Fällung mit Polyethylenglycol 6000 (PEG) (Pasteur Diagnostici, Mailand Italien) sowohl mit dem herkömmlichen als auch mit dem modifizierten Verfahren am Plasma von 250 Personen vorgenommen, die sich ins Laboratorium begeben hatten. Dabei wurden folgende Resultate erhalten:

$$r = 0,977 \quad tStudent = 17,384 \quad p = < 0,00001$$

Die beim herkömmlichen Verfahren vorgenommene Abänderung besteht im wesentlichen darin, entnommenes Vollblut unmittelbar in einem Probeglas aufzunehmen, das einen negativen Innendruck aufweist und, neben dem üblichen Harz und gerinnungshemmenden Wirkstoff, eine vorher bestimmte Menge von PEG oder anderen, Fraktionen des Blutcholesterins niederschlagenden Substanzen enthält.

Im weiteren Verlauf der Analyse ermöglicht diese Massnahme eine Einsparung von Verfahrensschritten (zwei Saugschritte, ein Schleuderschritt und ein Schüttelschritt), sowie eine Materialersparnis (zwei Stützen, ein Probeglas und ein Probebecher).

<u>Beispiel für die Vorbereitung des Vakuum-Probeglases unter Verwendung von PEG als Fraktionen des Blutcholesterins niederschlagende Substanz.</u>

Da das herkömmliche Verfahren ein Verhältnis 1:1 vom PEG zum Plasma vorsieht, wurde diejenige Menge PAG berechnet, die, eingebracht in das Probeglas vor der Blutentnahme, das gleiche Verhältnis PEG/Plasma nach dem Schleudern ergibt.

Vorausgeschickt, dass der Mittelwert des Hämatokryts einer Auswahl von 950 Personen, für die eine Bestimmung des HDL-Cholesterins verlangt wurde, sich auf 0,407 belief und dass das verwendete Probeglas 0,25 ml Harz und Lithium-Heparin enthält, wurden folgende Formeln angewendet:

$$A \quad 2,50 \text{ ml} - 0,25 \text{ ml} = 2,25 \text{ ml}$$

zur Aufnahme von Blut + PEG bestimmter Rauminhalt des Probeglases

$$B \quad 2,25 \text{ ml} = x + 0,593x$$

wobei x das Volumen des Vollblutes und 0,593x das Volumen des entsprechenden Plasmas (und damit auch des PEGs) für ein 0,407 betragendes Hämatokryts darstellt, so dass

$$C \quad x = \frac{2,25}{1,593} \text{ ml} = 1,41 \text{ ml}.$$

Berücksichtigt man, dass der Mittelwert des Hämatokryts (= 0,407) das Ergebnis einer die Erscheinung des "trapped Plasmas" nicht in Erwägung ziehenden elektronischen Zählung ist und dass die nach dem Schleudern eines Probeglases mit einem Durchmesser von 10 mm (Makrohämatokryt) in Wirklichkeit erhaltene Plasmamenge um 7 Hundertstel geringer ist, wird die Formel B wie folgt abgeändert:

$$B' \quad 2,25 \text{ ml} = x + 0,523x$$

daher wird

$$C' \quad x = \frac{2,25}{1,523} \text{ ml} = 1,48 \text{ ml}$$

daraus folgt

$$D \quad 2,25 \text{ ml} = 1,48 \text{ ml} + 0,77 \text{ ml}$$

wobei 0,77 ml = Volumen des PEGs.

Die Anwendung des zur Durchführung der Dosierung des HDL-Cholesterins vorgeschlagenen Vakuum-Probeglases bietet die Erzielung mit dem herkömmlichen Verfahren übereinstimmender Resultate und eine Reihe unbestreitbarer Vorteile:

1. Vermeidung der sonst bei der Überführung des Plasmas vom Probeglas A in das Probeglas B nach dem herkömmlichen Verfahren möglichen Personenverwechslung;

2. Verbesserung der Hygiene und der Gesundheitswahrung der Laboranten zumal beim abgeänderten Verfahren die gesamten Verfahrensschritte, im Gegensatz zum herkömmlichen Verfahren, in einem geschlossenen System durchführbar sind;

3. Zeitersparnis (ca. 40 Minuten) und Materialersparnis (zwei Stützen, ein Probeglas und ein Probebecher);

4. Möglichkeit, die Untersuchung zu wiederholen bzw. um 24 Stunden aufzuschieben (Lagerfähigkeit des Probeglases auch bei Raumtemperatur), was mit dem bisherigen Verfahren unmöglich war, da ca. 60 Minuten nach dem Schleudern der Inhalt des Probeglases B zu einem Gel wird, welches das Saugen und damit auch die Analyse nicht mehr zulässt, wogegen dies in dem beim abgeänderten Verfahren eingesetzten Probeglas auch nach dem Schleudern nicht vorkommt.

Das erfindungsgemässe Verfahren lässt sich daher wie folgt schematisch darstellen:

PROBEENTNAHME
↓
PROBEGLAS (A) enthaltend FRAKTIONEN DES BLUTCHOLESTERINS NIEDERSCHLAGENDE SUBSTANZ(en)
↓
SCHLEUDERN
↓
A N A L Y S E

## Patentansprüche

1. Verfahren zur Bestimmung des HDL-Cholesterins mit gegenüber dem Luftdruck einen negativen Innendruck aufweisendem Probeglas, dadurch gekennzeichnet, dass ein und dasselbe Probeglas sowohl zur Probeentnahme, als auch zur Analyse verwendet wird.

2. Bei einem Verfahren nach Anspruch 1 verwendetes Probeglas, dadurch gekennzeichnet, dass das Probeglas eine entsprechende. Menge einer oder mehrerer, Fraktionen des Blutcholesterins mittels Schleudern niederschlagender Substanzen enthält und als primäres Probeglas verwendbar ist.

Europäisches Patentamt

# EUROPÄISCHER RECHERCHENBERICHT

Nummer der Anmeldung

EP 95107934.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.6) |
|---|---|---|---|
| P,A | PATENT ABSTRACTS OF JAPAN, unexamined applications, P Sektion, Band 18, Nr. 412, 2. August 1994 THE PATENT OFFICE JAPANESE GOVERNMENT Seite 75 P 1780; & JP-A-06 123 741 (JII II DE ENG K.K.) ---- | 1,2 | G 01 N 33/92 |

RECHERCHIERTE SACHGEBIETE (Int Cl.6)

G 01 N 33/00

Der vorliegende Recherchenbericht wurde für alle Patentansprüche erstellt.

| Recherchenort | Abschlußdatum der Recherche | Prüfer |
|---|---|---|
| VIENNA | 18-08-1995 | SCHNASS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82